# EUROPEAN PATENT SPECIFICATION

(11) **EP 1 990 420 B1**
(45) Date of publication and mention of the grant of the patent: **11.08.2010**
(21) Application number: 07009398.4
(22) Date of filing: 10.05.2007
(51) Int. Cl.: C12Q 1/37

(54) **Method for the detection of the in-vivo activity of neurotrypsin, use of the method and use of the C-terminal 22-kDa fragment of agrin as biomarker in diagnosis and monitoring of neurotrypsin-related disturbances**
Verfahren zur Detektion der in-vivo-Aktivität von Neurotrypsin, Verwendung des Verfahrens und Verwendung des C-terminal 22-kDa-Fragments als Biomarker bei der Diagnose und Überwachung von Störungen im Zusammenhang mit Neurotrypsin
Procédé de détection d'une activité in-vivo de neurotrypsine, utilisation du procédé et utilisation du fragment 22-kDa du terminal C d'agrine comme un biomarqueur dans le diagnostic et la surveillance des perturbations liées à la neurotrypsine

(43) Date of publication of application: 12.11.2008
(73) Proprietor: Neurotune AG, 8952 Schlieren (CH); UNIVERSITY OF ZURICH, 8006 Zürich (CH)
(72) Inventor: Sonderegger, Peter, 8005 Zürich (CH); Hettwer, Stefan, 8051 Zürich (CH); Stephan, Alexander, 8037 Zürich (CH); Miyai, Kazumasa, 8004 Zürich (CH); Kunz, Beat, 8400 Winterthur (CH)
(74) Representative: Emmel, Thomas

(56) References cited:
- WO-A-2006/103261
- WO-A-2007/011682
- GESEMANN M ET AL: "Acetylcholine receptor-aggregating activity of agrin isoforms and mapping of the active site." THE JOURNAL OF CELL BIOLOGY FEB 1995, vol. 128, no. 4, February 1995 (1995-02), pages 625-636, XP002440442 ISSN: 0021-9525
- DATABASE MEDLINE [Online] US NATIONAL LIBRARY OF MEDICINE (NLM), BETHESDA, MD, US; October 2005 (2005-10), THADIKKARAN LYNNE ET AL: "The role of proteomics in the assessment of premature rupture of fetal membranes." Database accession no. NLM15970282 & CLINICA CHIMICA ACTA; INTERNATIONAL JOURNAL OF CLINICAL CHEMISTRY OCT 2005, vol. 360, no. 1-2, October 2005 (2005-10), pages 27-36, ISSN: 0009-8981

### SEQUENCE LISTING

<110> Neurotune AG Universität Zürich
<120> Method for the detection of the in-vivo activity of neurotrypsin, use of the method....
<130> 04398
<160> 15
<170> PatentIn version 3.1
<210> 1
   <211> 2045
   <212> PRT
   <213> Homo sapiens
<400> 1
<210> 2
   <211> 191
   <212> PRT
   <213> Homo sapiens
<400> 2
   Gly Leu Val Glu Lys Ser Ala Gly Asp Val Asp Thr Leu Ala Phe Asp
   1               5                   10                  15
   Gly Arg Thr Phe Val Glu Tyr Leu Asn Ala Val Thr Glu Ser Glu Lys
               20                  25                  30
   Ala Leu Gln Ser Asn His Phe Glu Leu Ser Leu Arg Thr Glu Ala Thr
           35                  40                  45
   Gln Gly Leu Val Leu Trp Ser Gly Lys Ala Thr Glu Arg Ala Asp Tyr
       50                  55                  60
   Val Ala Leu Ala Ile Val Asp Gly His Leu Gln Leu Ser Tyr Asn Leu
   65                  70                  75                  80
   Gly Ser Gln Pro Val Val Leu Arg Ser Thr Val Pro Val Asn Thr Asn
                   85                  90                  95
   Arg Trp Leu Arg Val Val Ala His Arg Glu Gln Arg Glu Gly Ser Leu
               100                 105                 110
   Gln Val Gly Asn Glu Ala Pro Val Thr Gly Ser Ser Pro Leu Gly Ala
           115                 120                 125
   Thr Gln Leu Asp Thr Asp Gly Ala Leu Trp Leu Gly Gly Leu Pro Glu
       130                 135                 140
   Leu Pro Val Gly Pro Ala Leu Pro Lys Ala Tyr Gly Thr Gly Phe Val
   145                 150                 155                 160
   Gly Cys Leu Arg Asp Val Val Val Gly Arg His Pro Leu His Leu Leu
                   165                 170                 175
   Glu Asp Ala Val Thr Lys Pro Glu Leu Arg Pro Cys Pro Thr Pro
               180                 185                 190
<210> 3
   <211> 13
   <212> PRT
   <213> Homo sapiens
<400> 3
   Ser Ala Gly Asp Val Asp Thr Leu Ala Phe Asp Gly Arg
   1               5                   10
<210> 4
   <211> 14
   <212> PRT
   <213> Homo sapiens
<400> 4
   Thr Phe Val Glu Tyr Leu Asn Ala Val Thr Glu Ser Glu Lys
   1               5                   10
<210> 5
   <211> 15
   <212> PRT
   <213> Rattus rattus
<400> 5
   Ala Ser Cys Tyr Asn Ser Pro Leu Gly Cys Cys Ser Asp Gly Lys
   1               5                   10                  15
<210> 6
   <211> 10
   <212> PRT
   <213> Rattus rattus
<400> 6 <210> 7
   <211> 8
   <212> PRT
   <213> Rattus rattus
<400> 7
<210> 8
   <211> 8
   <212> PRT
   <213> Rattus rattus
<400> 8
   Leu Val Glu Lys Ser Val Gly Asp
   1               5
<210> 9
   <211> 62
   <212> DNA
   <213> Artificial sequence
<220>
   <223> Primer
<400> 9
   gcgcgagtta accaccatca ccatcaccat caccattcag cgggggacgt ggataccttg 60
   gc 62
<210> 10
   <211> 41
   <212> DNA
   <213> Artificial Sequence
<220>
   <223> Primer
<400> 10
   ttacctgcgg ccgctcatgg ggtggggcag ggccgcagct c 41
<210> 11
   <211> 12
   <212> PRT
   <213> Artificial Sequence
<220>
   <223> His-Tag
<400> 11
   Ala Arg Val Asn His His His His His His His His
   1 5 10
<210> 12
   <211> 186
   <212> PRT
   <213> Homo sapiens
<400> 12
   Ser Ala Gly Asp Val Asp Thr Leu Ala Phe Asp Gly Arg Thr Phe Val
   1 5 10 15
   Glu Tyr Leu Asn Ala Val Thr Glu Ser Glu Lys Ala Leu Gln Ser Asn
   20 25 30
   His Phe Glu Leu Ser Leu Arg Thr Glu Ala Thr Gln Gly Leu Val Leu
   35 40 45
   Trp Ser Gly Lys Ala Thr Glu Arg Ala Asp Tyr Val Ala Leu Ala Ile
   50 55 60
   Val Asp Gly His Leu Gln Leu Ser Tyr Asn Leu Gly Ser Gln Pro Val
   65 70 75 80
   Val Leu Arg Ser Thr Val Pro Val Asn Thr Asn Arg Trp Leu Arg Val
   85 90 95
   Val Ala His Arg Glu Gln Arg Glu Gly Ser Leu Gln Val Gly Asn Glu
   100 105 110
   Ala Pro Val Thr Gly Ser Ser Pro Leu Gly Ala Thr Gln Leu Asp Thr
   115 120 125
   Asp Gly Ala Leu Trp Leu Gly Gly Leu Pro Glu Leu Pro Val Gly Pro
   130 135 140
   Ala Leu Pro Lys Ala Tyr Gly Thr Gly Phe Val Gly Cys Leu Arg Asp
   145 150 155 160
   Val Val Val Gly Arg His Pro Leu His Leu Leu Glu Asp Ala Val Thr
   165 170 175
   Lys Pro Glu Leu Arg Pro Cys Pro Thr Pro
   180 185
<210> 13
   <211> 8
   <212> PRT
   <213> Artificial Sequence
<220>
   <223> Amino Acid Insert
<400> 13
   Glu Leu Ala Asn Glu Ile Pro Val
   1 5
<210> 14
   <211> 11
   <212> PRT
   <213> Artificial sequence
<220>
   <223> Amino Acid Insert
<400> 14
   Pro Glu Thr Leu Asp Ser Gly Ala Leu His Ser
   1 5 10
<210> 15
   <211> 19
   <212> PRT
   <213> Artificial Sequence
<220>
   <223> Amino Acid Insert
<400> 15
   Glu Leu Ala Asn Glu Ile Pro Val Pro Glu Thr Leu Asp Ser Gly Ala
   1               5                   10                  15
   Leu His Ser

## Description

### FIELD OF THE INVENTION:

The invention relates to a method for the detection of the in-vivo activity of neurotrypsin, the use of such method and the use of the C-terminal 22-kDa fragment of agrin as biomarker in different diagnostic or clinical applications all directly or indirectly related to the activity of the enzyme neurotrypsin.

Biomarkers report about biological phenomena ongoing within a disease or treatment. A biomarker is defined as any characteristic that can be objectively measured and evaluated as an indicator of a normal or pathological biological process or of a pharmacological response to a therapeutic intervention. Distinct types of biomarkers report about molecular characteristics, physiological parameters (for example blood pressure, heart rate), or they provide imaging information. Importantly, they provide information about an "outcome": e.g. about the result of a molecular, cellular, or physiological mechanism, about a therapeutic benefit, or about the risk of a therapeutic intervention. Useful biomarkers should satisfy two criteria, they must be associated with the biological mechanisms ongoing within a disease or treatment, and they must correlate statistically with the clinical outcome.

Neurotrypsin is a trypsin-like serine protease. It shows a unique domain composition (Proba et al., 1998). It consists of a proline-rich basic segment, one kringle domain, four scavenger receptor cysteine-rich (SRCR) domains, and a protease domain.

Neurotrypsin is predominantly expressed in neurons of the cerebral cortex, the hippocampus and the amygdala (Gschwend et al., 1997). By immuno-electronmicroscopy, neurotrypsin was localized in the presynaptic membrane and the presynaptic active zone of both asymmetrical (excitatory) and symmetrical (inhibitory) synapses (Molinari et al., 2002).

Neurotrypsin plays an important role in neuropsychiatric disturbances as well as in disturbances outside the nervous system.

Recently, neurotrypsin was identified as a cause of a severe autosomal-recessive form of mental retardation (Molinari et al., 2002). Individuals suffering from neurotrypsin-dependent mental retardation show a 4-bp deletion in the 7th exon of the neurotrypsin gene resulting in a shortened protein lacking the catalytic domain. The pathophysiological phenotype and the age of onset of the disease in affected individuals characterizes neurotrypsin as a regulator of adaptive synaptic functions, such as synapse reorganization during later stages of neurodevelopment and adult synaptic plasticity. After normal psychomotor development in the first 18 months, the affected individuals showed first signs of mental retardation when they were around 2 years of age. This indicates that neurotrypsin function is crucial in later stages of brain development as e.g. for adaptive synaptic functions required to establish and/or maintain higher cognitive functions.

In WO 2006/103261 is shown that neurotrypsin overexpression in motoneurons of transgenic mice results in a degeneration of the neuromuscular junction, which in turn causes the death of the denervated muscle fibers. Muscle fiber loss is characteristic for the type of muscle atrophy found in elderly humans, termed sar-copenia. Therefore, it has been postulated that the inhibition of neurotrypsin could have a beneficial effect on age-dependent muscle fiber denervation, muscle fiber loss, and skeletal muscle atrophy.

Further data (Aimes et al., 2005) suggest a potential role for serine proteases, like neurotrypsin in vascular function and angiogenesis.

In view of the central role of neurotrypsin in metabolism, it is desirable to determine and monitor neurotrypsin related disturbances in patients, or to assess the effect of medicaments on the neurotrypsin status *in vivo,* respectively.

### SUMMARY OF THE INVENTION

The inventors have found out that the object of the invention can be realized by a method according to claim 1, in which the 22 kDa fragment of agrin is measured in blood or urine in order to determine the in-vivo activity of neurotrypsin and by the use of such method according to claim 3 for diagnosis and monitoring of neurotrypsin related disturbances. The invention further covers the use of the 22 kDa fragment of agrin as special biomarker according to claim 8.

A Further independent claim is directed to the use of the 22-kDa-fragment of agrin as biomarker in clinical or preclinical studies to establish the effect of substances on the activity of neurotrypsin,

Preferred embodiments of the invention are addressed to in the subclaims.

### BRIEF DESCRIPTION OF THE FIGURES

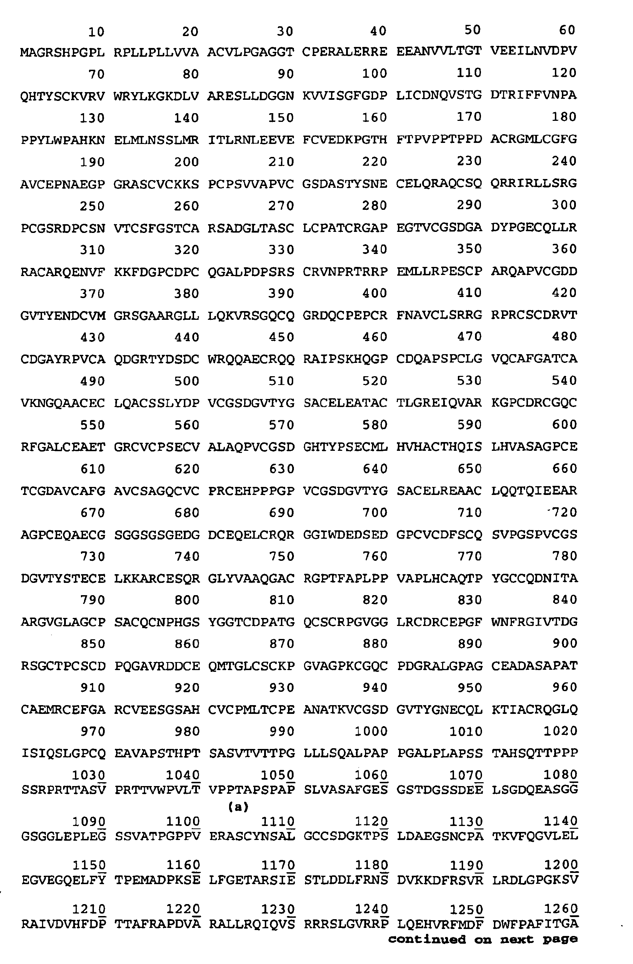

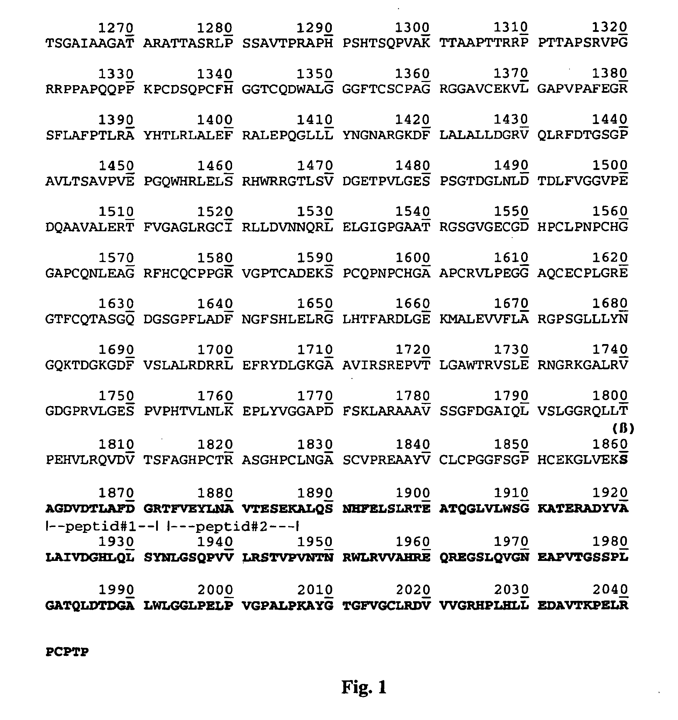

Fig. 1 shows the protein sequence of human agrin (SEQ ID NO: 1). The whole length of the unprocessed precursor is 2045 amino acids. In the sequence portion shown the position of α- and β-cleavage sites are marked. Furthermore some subsequences referred to in the examples are marked. The sequence of the 22-kDa fragment of agrin is shown in bold types.

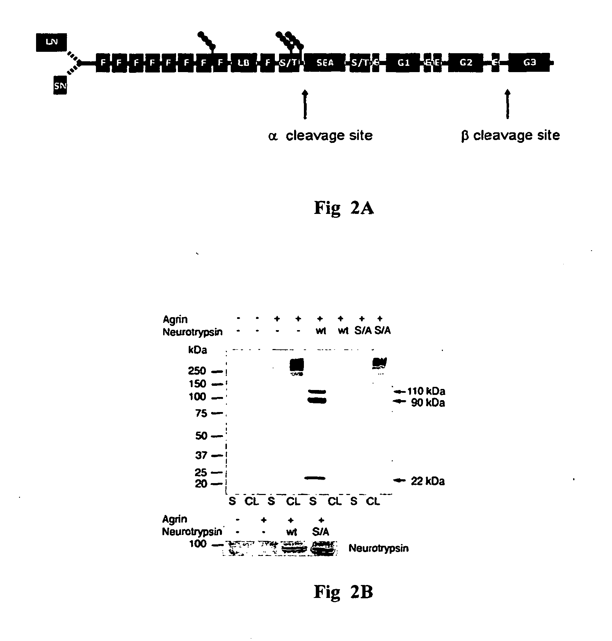

Fig. 2A is a schematic representation of the domain organization of agrin and localization of its neurotrypsin-dependent cleavage sites. Agrin has a core protein mass of approximately 220 kDa. It is a multidomain protein composed of 9 FS (follistatin-like) domains, 2 LE (laminin-EGF-like) domains, one SEA (sperm protein enterokinase and agrin) domain, 4 EG (epidermal growth factor-like) domains, and 3 LG (laminin globular) domains. On both sides of the SEA domain an S/T (serine/threonine-rich) region is found.

Agrin exists in several isoforms, e.g. a secreted isoform with an N-terminal agrin (NtA) domain and a type II transmembrane isoform with an N-terminal transmembrane (TM) segment securing its anchorage in the plasma membrane. Splice variants of the C-terminal moiety: A 4 amino acid-long insert at the A/y site, and the three different inserts at the B/z site composed of 8, 11 or 19 (8+11) amino acids. Neurotrypsin cleaves agrin at two sites. One site (termed α site) is located between arginine 1102 (R1102) and alanine 1103 (A1103). The other site (termed β site) is located between lysine 1859 (K1859) and serine 1860 (S1860). Amino acid numbers refer to secreted agrin (splice variant A0B0) of the human ortholog (UniProtKB/Swiss-Prot 000468). Agrin cleavage by neurotrypsin generates an N-terminal fragment in the ranges of 110-400 kDa (due to differential carbohydrate levels), a middle fragment of approx. 90 kDa and a C-terminal fragment of 22 kDa.
Fig. 2B shows how neurotrypsin cleaves agrin *in vitro.* Western blot analysis of HeLa cells cotransfected with combinations of membrane-bound agrin (+), wild-type neurotrypsin (wt), inactive neurotrypsin (S/A), and empty pcDNA3.1 (-).
   The supernatants (S) and cell lysates (CL) were analyzed with anti-agrin antibodies directed against the C-terminus of agrin. Upper panel: Transfection of agrin alone resulted in a signal above 250 kDa in the cell lysate. Upon cotransfection with wild-type neurotrypsin, full-length agrin was cleaved resulting in fragments running at 22, 90, and 110 kDa in the supernatant. No cleavage was found after cotransfection with inactive neurotrypsin. Lower panel: Control for neurotrypsin expression with antibodies against neurotrypsin.

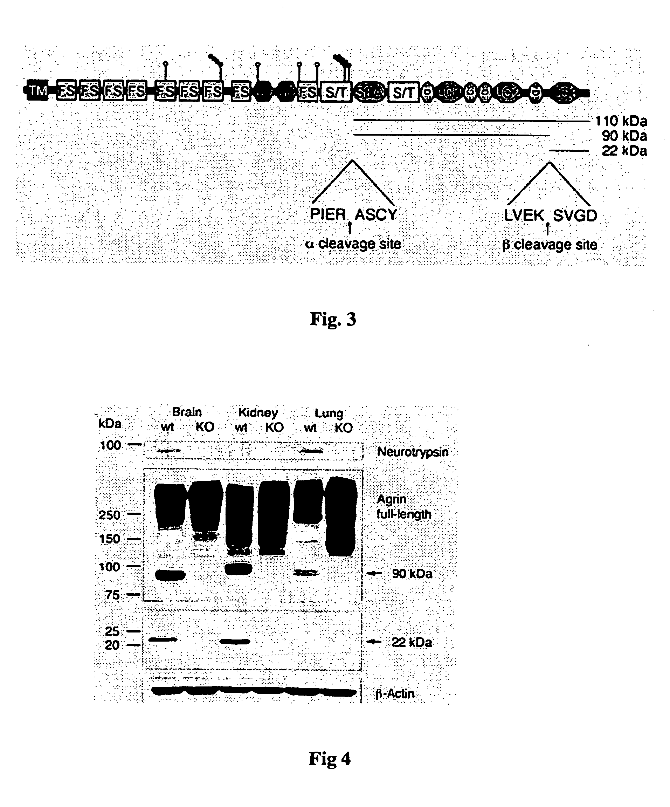

Fig. 3 shows domain structure of transmembrane agrin. The sequences of the α-and β-cleavage sites and the C-terminal fragments resulting from neurotrypsin cleavage are indicated. TM, transmembrane segment; FS, cysteine-rich repeat similar to follistatin; LE, laminin EGF-like domain; S/T, serine/threonine-rich region; SEA, sperm protein, enterokinase, and agrin domain; EG, epidermal growth factor (EGF)-like domain; LG, laminin globular domain; single circles, sites of N-linked glycosylation; multiple circles, sites of glycan attachment.
Fig. 4 shows that neurotrypsin cleaves agrin *in vivo.* Western blot analysis of tissue from wild-type (wt) and neurotrypsin-deficient (KO) mice. The 90-kDa agrin cleavage product was detected in brain, kidney, and lung of wild-type mice, but was absent in neurotrypsin-deficient. Similar results were obtained for the 22-kDa agrin fragment except for the lung. β-Actin was used as a loading control.

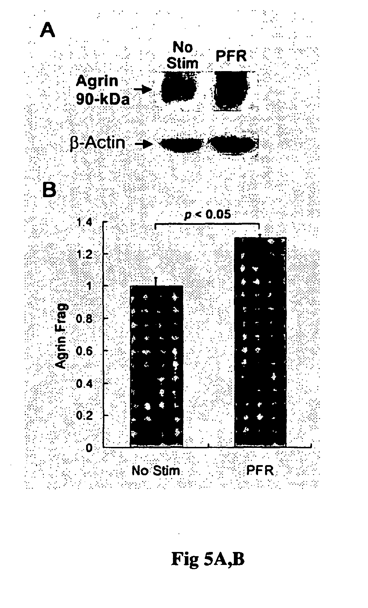

Fig. 5 A, B show that chemical stimulation of long-term potentiation induces an increase in proteolytic activity of neurotrypsin.
Fig. 5 A is a Western blotting for agrin (upper panel) and β-actin (lower panel) in hippocampal slices without stimulation (No Stim) and stimulated by the combination of picrotoxin, forskolin, and rolipram (PFR). The signal intensity of the 90-kDa fragment of agrin cleaved by neurotrypsin in PFR-stimulated hippocampus is more intense than in the non-stimulated control.
Fig. 5 B shows the ratio of the signal intensity of the 90-kDa fragment of agrin normalized by β-actin signal intensity. The average of the signal intensities in the non-stimulated controls was set to 1. Three independent experiments showed a significant 30% increase of the agrin fragment by PFR stimulation compared to the non-stimulated controls (p < 0.05 by Student's t-test).

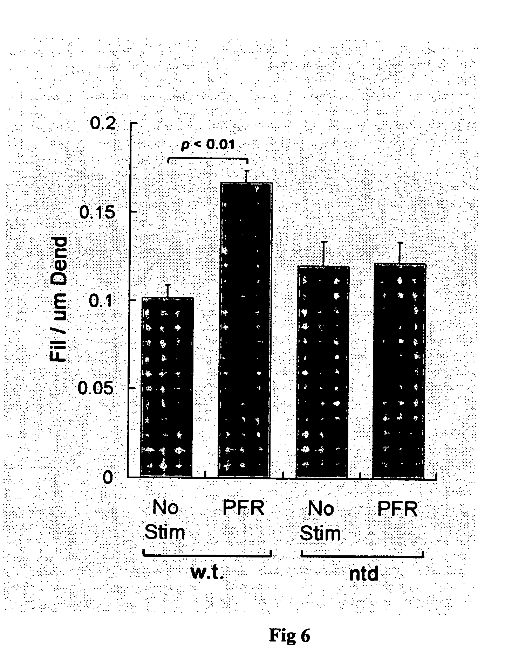

Fig. 6 demonstrates how chemical stimulation of long-term potentiation increases the number of filopodia in a neurotrypsin-dependent manner.

The histogram shows the numbers of filopodia (Fil) per 1 µm of secondary apical dendrite (Dend) of hippocampal CA1 pyramidal neurons under four different conditions. In wild-type mice (w.t.), stimulation by a combination of picrotoxin, forskolin, and rolipram (PFR) induced a significant increase in filopodia number in comparison to the non-stimulated controls (No Stim; p < 0.01 by Student's t-test). In contrast, neurotrypsin-deficient mice (ntd) showed no significant change in filopodia number by PFR stimulation.

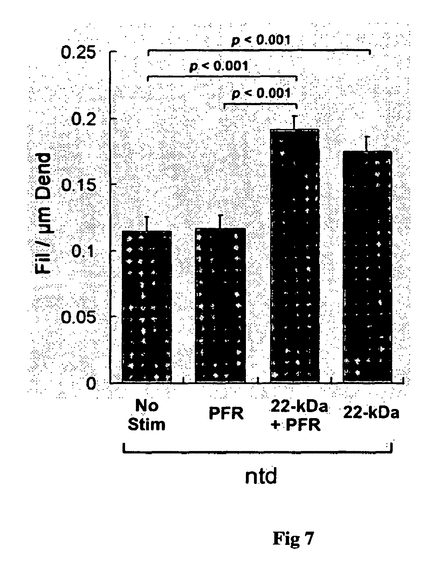

Fig.7 is a histogram showings the numbers of filopodia (Fil) per 1 µm of secondary apical dendrite (Dend) of hippocampal CA1 pyramidal neurons in neurotrypsin-deficient mice (ntd). PFR stimulation alone (PFR) did not induce a significant increase in filopodia number in comparison to non-stimulated controls (No Stim). Co-administration of picrotoxin, forskolin, and rolipram (PFR) and 22-kDa fragment (PFR+22-kDa) rescued the chemical stimulation-induced increase in filopodia number (p < 0.001 vs No Stim and PFR by Student's t-test). In addition, 22-kDa fragment without PFR stimulation (22-kD frag) also induced a significant increase in filopodia number (p < 0.001 vs No Stim by Student's t-test).

Hence the 22-kDa fragment of agrin induces an increase in filopodia number in neurotrypsin-deficient mice.

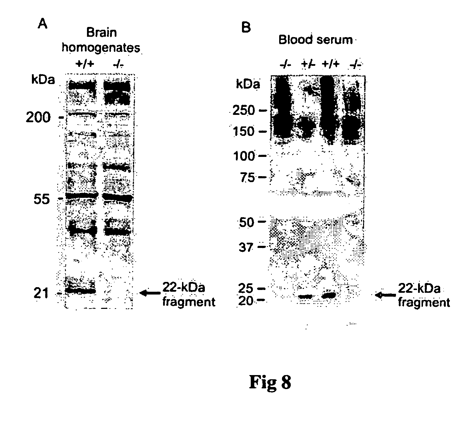

Fig. 8A is a Western blot analysis of the brain homogenate of a wild-type (+/+) and a neurotrypsin-deficient (-/-) mouse. Note that the 22-kDa fragment of agrin is only detected in the wild-type mouse. This indicates that the 22-Da fragment is indeed the result of neurotrypsin-dependent cleavage of agrin.
Fig. 8B is a Western blot of blood serum of a wild-type (+/+), a homozygeous neurotrypsin-deficient (-/-), and a heterozygeous neurotrypsin-deficient (+/-) mouse. It is shown that the 22-kDa fragment of agrin is only present in mice expressing functional neurotrypsin (+/+ and +/-).

Additionylly from both Figs. 8 A, B it is evident that the 22-kDa fragment of agrin is present in the brain homogenate and the blood of the mouse.

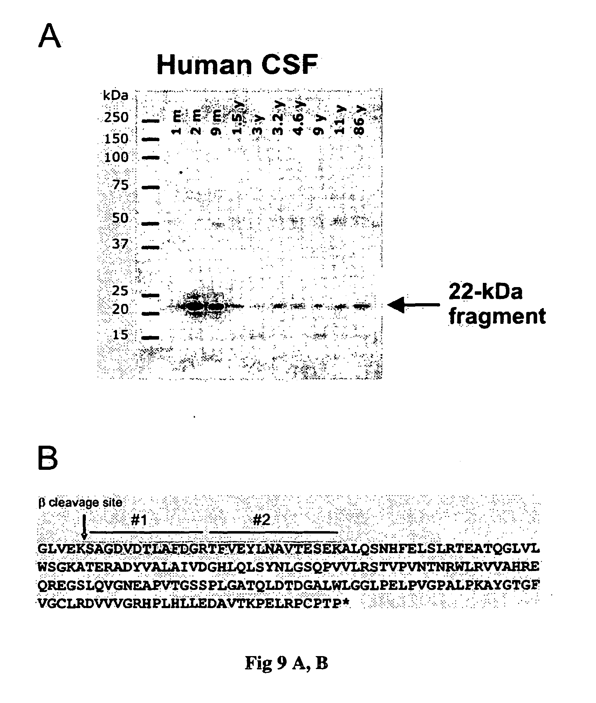

Fig.9A is a Western blotting of CSF samples of humans in the age-range between 1 month and 86 years. In each lane, 10 µl human CSF were analyzed. A commercially available gradient SDS-PAGE gel (NuPAGE, 4-12 %) was used. Immunodetection was performed with the antibody R139 against the 22-kDa fragment of agrin. In accordance with the elevated expression level of neurotrypsin during neural development, the neurotrypsin-dependent 22-kDa fragment of agrin is most abundant at the ages of 2 and 9 months.
Fig 9B is a LC/MS analysis of the 22-kDa fragment found in human CSF. The presented sequence comprises the 22-kDa fragment and the five amino acids preceeding the β cleavage site (SEQ ID NO: 2). CSF samples of several individuals were pooled and their proteins chromatographically separated in order to enrich for the 22-kDa fragment identified by Western blotting. The fraction enriched for the 22-kDa fragment was cut out of a SDS-PAGE gel and subjected to LC/ESI/MS/MS analysis. Two peptides, peptide #1 (SEQ ID NO: 3) and #2 (SEQ ID NO: 4), were identified. Both were localized to the 22-kDa fragment of agrin.
   Both Figs. 9A, B show that the 22-kDa fragment of agrin can be detected in human cerebrospinal fluid (CSF).

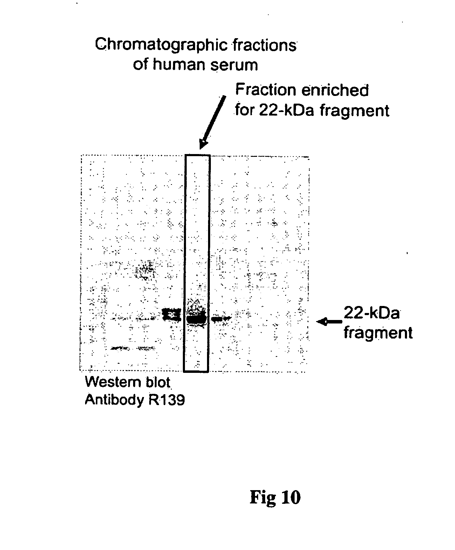

Fig. 10 is a Western blot of chromatographically processed human blood serum showing a strong band immunoreactive for the 22-kDa fragment of agrin. Final confirmation of its identity will require MS analysis, however, the present results are a strong piece of evidence that the 22-kDa fragment of agrin can be found in human blood serum.

### DESCRIPTION

The C- terminal 22-kDa fragment of the proteoglycan agrin used in the method of the invention and as biomarker, is one naturally generated product of the proteolytic cleavage of agrin by its unique processing enzyme neurotrypsin.

Agrin is a well characterized molecule (Bezakova et al., 2003; Sanes and Lichtman, 2001). It has a core protein mass of approximately 220 kDa. Agrin exists in several isoforms and splice variants

As is illustrated in Fig. 2A, agrin is cleaved by the serine protease neurotrypsin at two homologous sites, termed α- and β-cleavage site. The cleavage of agrin by neurotrypsin resulting from coexpression of the two proteins in HeLa cells liberates three fragments of agrin into the culture supernatant (Example 1 and Fig. 1B). The 22-kDa fragment ranges from the β-cleavage site to the C-terminus of agrin. The 90-kDa fragment spans from the α- to the β- cleavage site, while the 110-kDa fragment ranges from the α-cleavage site to the C-terminus and, thus, is the result of incomplete cleavage at the β-site. The N-terminal fragment remains with the cells and is probably degraded rapidly via endocytosis.

Both cleavage sites were unequivocally identified and found to be homologous and conserved in evolution (Example 2).

The term 22-kDa fragment of agrin if used in this application spans from the β-cleavage site to the C-terminus of agrin and shall encompass all different isoforms and splice variants of the fragment occuring *in vivo.* In some publications, the C-terminal laminin G domain (LG3) of agrin is referred to as 20-kDa fragment. The protein meant by this term is strucutrally equivalent to the fragment used according to the invention, but it has been generated artificially as a recombinant protein corresponding to the C-terminal LG domain of agrin for the purpose of testing its function. In contrast, it is an essential aspect that the C-terminal LG domain of agrin is naturally separated from the rest of the agrin molecule by neurotrypsin-dependent proteolytic cleavage. Only after proteolytic cleavage at the so-called β cleavage site, the 22-kDa fragment of agrin becomes mobile and translocates from the site of its generation into the cerebrospinal fluid or the blood, where it can be detected based on established detection methods and, thus, serve as a biomarker.

From the WO 2006/103261 cited above a test method is known for determining the effect of inhibiting substances on the activity of neurotrypsin. A typical protocol of the known method provides that the inhibiting substance is incubated together with neurotrypsin and agrin and the amount of cleavage of agrin is determined based on measurement of fragments generated by the proteolytic activity of neurotrypsin.

The known method refers to an *in vitro* enzyme assay with a limited number of reaction partners. As the situation *in vivo* is far more complex it could not be expected that a special fragment of agrin could also be found in the CSF or the blood and used as an *in vivo* biomarker, in order to report about *in vivo* activity of neurotrypsin in the brain and in extraneural tissues, respectively. First, from *in vitro* cleavage of a protein or peptide it can not be concluded that the cleavage also occurs *in vivo. In vitro* cleavage is an artificial experimental reaction. It is achieved by putting the proteolytic enzyme and a protein together in a test tube and incubating them together to allow for the cleavage reaction to occur. *In vivo* cleavage of a protein or peptide, in contrast, requires that it is colocalized with a given proteolytic enzyme in space and time, in order for the reaction to take place. For example, it can be demonstrated *in vitro* that agrin can be cleaved by the pancreatic serine protease trypsin. However, trypsin can not cleave agrin *in vivo*, because agrin is not colocalized *in vivo* with trypsin. The inventors found that selective inactivation of neurotrypsin by gene targeting abolishes agrin cleavage completely (Example 3). This indicates that only neurotrypsin can cleave agrin *in vivo,* while other trypsin-like serine proteases, such as trypsin, which can cleave agrin *in vitro,* do not cleave agrin *in vivo.* Second, even from the fact that *in vivo* cleavage of agrin by neurotrypsin is found in homogenized tissue, it can not be predicted, whether a neurotrypsin-dependent fragment of agrin will be found in the CSF or the blood. In CSF and blood, full-length agrin and the 90-kDa fragment are only found in neglectable amounts. Neurotrypsin is not detectable in the CSF and the blood. Therefore, the cleavage of agrin by neurotrypsin does neither occur in the CSF nor in the blood, but in the interior of the neural and non-neural tissues where agrin and neurotrypsin encounter on cell surfaces or in the extracellular matrix. Therefore, the occurrence of the 22-kDa fragment in CSF and blood is definitely an unprecedented and non-trivial observation. In support of this conclusion, it may be noted that neither the N-terminal fragment nor the middle 90-kDa fragment of agrin show up in CSF or blood after their generation by neurotrypsin. Therefore, of the three fragments of agrin that are generated by neurotrypsin's proteolytic activity only the 22-kDa fragment is efficiently translocated into CSF and blood, while the other two fragments are not.

The occurrence of the 22-kDa fragment of agrin in the CSF and the blood reflects the level of neurotrypsin activity in the brain and in non-neural tissues, respectively.

With reference to neurotrypsin activity in the brain, the inventors were the first to show that brain homogenates of wild-type mice contain the 22-kDa fragment of agrin, which in contrast is not found in brain homogenates of neurotrypsin-deficient mice (Example 3). Furthermore, the inventors were able to demonstrate the presence of the 22-kDa fragment of agrin in preparations of isolated synapses, called synaptosomes. This observation indicates that brain-derived 22-kDa fragment of agrin originates from synapses, where neurotrypsin is secreted and encounters agrin in the extracellular space. After local cleavage of agrin at the synapse or in the vicinity of the synapse, the 22-kDa fragment selectively translocates to the CSF. Therefore, the level of the 22-kDa fragment of agrin in the CSF reports about neurotrypsin activity at the CNS synapse.

With reference to neurotrypsin activity in non-neural tissues, the inventors showed that neurotrypsin is expressed in the kidney and the lung and that neurotrypsin-dependent cleavage of agrin in kidney and lung generates agrin fragments of 90 kDa and 22 kDa. Because both kidney and lung are highly vascularized tissues, it is very likely that at least part of the 22-kDa fragment of agrin that is found in the blood originates from the kidney and/or the lung. Therefore, the level of 22-kDa fragment found in the blood reports about neurotrypsin activity in kidney and/or lung.

A fraction of the 22-kDa fragment of agrin found in the blood very likely originates from the neuromuscular junction. The inventors found that motoneuron-derived neurotrypsin that is transported along the motor nerves to the skeletal muscles cleaves agrin at the neuromuscular junction. The 22-kDa fragment of agrin that is released at the NMJ most likely translocates into the blood. Therefore, the level of 22-kDa fragment of agrin found in the blood also reports on neurotrypsin activity at the NMJ.

The assessment of neurotrypsin activity in the brain or in extraneural tissues via measuring the levels of the 22-kDa fragment of agrin in the CSF and the blood, respectively, allows to draw conclusions about neurotrypsin-dependent physiological or pathological states.

The inventors recognized neurotrypsin as a promotor of synaptic plasticity in the CNS. They demonstrated that the promotion of synaptic plasticity by neurotrypsin depends on the cleavage of agrin, resulting in the generation and the release of the C-terminal 22-kDa fragment (Example 4). The 22-kDa fragment, in turn, has synapse-stimulating and thus plasticity-promoting activity. Using an experimental model system based on hippocampal tissue slices, the inventors found that filopodia promotion upon induction of long-term potentiation was abolished in neurotrypsin-deficient mice (Example 5). They also found that the 22-kDa fragment generated by neurotrypsin via cleavage of agrin is instrumental in the neurotrypsin-dependent promotion of synaptic plasticity via increasing the number of dendritic filopodia (Example 6). Dendritic filopodia are precursors of synapses. Increasing dendritic filopodia promotes reorganization of the synaptic circuitry in the CNS. Experiments with hippocampi of neurotrypsin-deficient mice showed that without the neurotrypsin-dependent generation of the 22-kDa fragment of agrin, activity-dependent promotion of dendritic filopodia is not functional. Hence a direct connection between a pathologic neurotrypsin-dependent status and the 22-kDa fragment of agrin could be established in an animal model. This observation in mice is in good accordance with the observation made in human individuals deficient in neurotrypsin indicating that adaptive synaptic plasticity is not functional without neurotrypsin and a strong deficiency in higher (cognitive) brain function, also called mental retardation, results (Molinari et al., 2002). Therefore, monitoring the activity of neurotrypsin at synapses via measuring the 22-kDa fragment of agrin in the CSF allows the diagosis of deregulations of neurotrypsin-dependent synaptic functions, such as neurotrysin-dependent synaptic plasticity.

In further experiments it was shown that the 22-kDa fragment of agrin can be detected in blood or CSF of mice and as well of human patients. Antibodies for the detection of the 22-kDa fragment of agrin are generated by first producing recombinant 22-kDa fragment of agrin (Example 7) and then use it for any one of the generally used methods for antibody production (Example 8).

Using antibodies against the 22-kDa fragment of agrin, its presence in brain homogenate and blood of the mouse (Example 9), as well as in human CSF (Example 10) and blood (Example 11) was demonstrated. This makes the fragment a suitable biomarker for routine applications.

Accordingly the invention encompasses a method for determining in vivo the activity of neurotrypsin by measuring the amount of the 22-kDa fragment of agrin in a patient sample. By using this method neurotrypsin-related disturbances can be diagnosed or monitored and in general the fragment can be used as biomarker for all neurotrypsin-related disturbances.

Such disturbances can especially be diseases caused by deregulation of neurotrypsin, where the term "deregulation" not only encompasses regulatory processes on the genetic level but also may reflect metabolic processes influencing the activity of neurotrypsin.

In a preferred example the agrin fragment is used as biomarker for diseases or disturbances of the neural or neuromuscular system related to neurotrypsin, such as neurotrypsin-dependent mental retardation and sarcopenia, the skeletal muscle atrophy of aged poeple, respectively.

The 22-kDa fragment of agrin can be used for diagnosing the disturbances or diseases mentioned. Apart from diagnosis it is also possible to use the biomarker in question for monitoring the trend or process of a disease connected to neurotrypsin.

As stated above it appears that a wide variety of diseases or disturbances can be connected to the enzyme neurotrypsin or its activity. One important therapeutic approach therefore is to find substances which influence, especially inhibit, the activity of neurotrypsin *in vivo.* Also in this context, i.e. in clinical or pre-clinical studies performed to determine the possible applicability of substances as e.g. neurotrypsin inhibitor, the 22-kDa fragment of agrin can be used as biomarker. The design of such studies does not represent a problem for a person skilled in the art. Any design is suited which enables the *in vivo* measurement of the 22-kDa fragment of agrin in patients who are treated with different substances selected for their possible action on neurotrypsin.

One main advantage in using the 22-kDa fragment of agrin as biomarker is that this fragment can be detected in blood or CSF which both are body fluids which can be analysed in routine applications. Detection of the fragment can be done by usual methods known in the art, like ELISA, immunoblot (Western Blot) techniques, RIA, flow cytometry, fluorescence polarization, latex agglutination, lateral flow assay, immunochromatographic assay, immunochips, dip stick immunotesting, or bead-based technology in combination with any other method (e.g. chemiluminescence, Luminex) to cite only some examples.

One main advantage of working with blood or CSF is that only the 22-kDa fragment of agrin is released after protolytic cleavage by neurotrypsin in the tissues mentioned. The whole agrin molecule is not or only to a negligible degree present in blood or CSF so that detection of the fragment in blood can be done with antibodies which not necessarily must differentiate between the whole agrin and its 22-kDa fragment.

Antibodies or other kinds of specific binding proteins which selectively bind to the 22 kDa fragment of agrin can be generated by anyone of the techniques known in the art (Roque et al., 2004; Gill and Damle, 2006). Preferably natural or recombinant antibodies or other specific binding proteins are generated by using a 22-kDa-fragment of agrin or a portion thereof prepared by recombinant techniques or chemical synthesis as a target.

The 22-kDa fragment of agrin as defined in the present invention is the 22 kDa fragment of human agrin of SEQ ID NO:12, which may further contain the 8, 11 or 19 (8+11) amino acids inserts of agrin isoforms at the B/z site. In human agrin the B/z site is located in the LG3 domain between amino acids Ser1884 and Glu1885 (Fig.1 and UniProtKB/Swiss-Prot 000468). The inserted sequences can be ELANEIPV (B/z 8; SEQ ID NO:13), PETLDSGALHS (B/z 11; SEQ ID NO:14) or ELANEIPVPETLDSGALHS (B/z 19; SEQ ID NO:15). In the present disclosure the term 22-kDa fragment of agrin also includes variants of the protein defined by SEQ ID NO:12 or the corresponding isoforms with 8, 11 or 19 additional amino acids, wherein one or more, in particular one, two, three or four amino acids are replaced by other amino acids, and/or wherein up to twelve amino acids are deleted either at the C-terminus or the N-terminus, or wherein up to 30 amino acids are added at the N-terminus. Such additional amino acids at the N-terminus are e.g. present due to the method of preparation by recombinant synthesis and expression in suitable cells. An example of such a protein falling under the definition of 22-kDa fragment of agrin is the protein prepared according to Example 7 of SEQ ID NO:12, which further contains an 8xHis tag with a glycine-serine (GS) linker to simplify purification and four additional amino acids at the N-terminal end. Proteins that are glycosylated or otherwise modified during expression in eukaryotic cells or otherwise are also included in the present definition of 22-kDa fragment of agrin.

Antibodies or other binding proteins directed against the 22-kDa fragment of agrin can also be generated by using only a portion of the 22-kDa fragment of agrin, for example a peptide of 18 amino acids with a sequence taken from the sequence of the 22-kDa fragment of agrin.

Additionally, it is covered by the invention that a 22-kDa-fragment of agrin prepared by recombinant techniques is used as a reference in the method according to claim 1 as a reference.

### EXAMPLES:

### Example 1: Cotransfection of agrin with neurotrypsin in eukaryotic cells results in agrin cleavage at two sites

To test agrin as a substrate for neurotrypsin HeLa cells were transfected with the transmembrane isoform of rat agrin (x4y8) alone or together with either wild-type neurotrypsin (wt) or an inactive form of neurotrypsin (S/A), in which the active site Ser was mutated to Ala.

For expression of neurotrypsin, the coding region of full-length human neurotrypsin (CAA04816) was inserted into the pcDNA3.1 vector (Invitrogen). Catalytically inactive neurotrypsin (S/A) was generated by mutating Ser₈₂₅ to Ala by means of overlap extension PCR. For expression of membrane-bound full-length agrin, the coding sequence ranging from Met₁ to Pro₁₉₄₈ of rat agrin (P25304, isoform 4, splice variant y4z8) was inserted into pcDNA3.1. HeLa cells were cultured to 60-80% confluency in 12-well culture plates (Corning) with DMEM supplemented with 10% FCS (Biochrom). Polyethylenimine (PEI) was used as transfection reagent as described in Baldi et al., 2005. The transfection mixture was removed 4-6 h after transfection by washing once with phosphate-buffered saline (PBS). After 48h in DMEM without FCS the medium was harvested and the cells were lysed in 150 mM NaCl, 0.5 mM EDTA, 10% glycerol, 1% Triton-X-100 in 20 mM Tris-HCl, pH 7.4. For the analysis of cleavage products supernatants and cell lysates were separated on a 4-12% NuPAGE gel (Invitrogen) and analyzed by immunodetection.

In lysates of cells transfected with agrin alone a smear above 250 kDa, characteristic for full-length agrin, was detected in Western blots with antibodies directed against its C-terminal moiety (Fig. 2A). The same signal was also found in lysates of cells cotransfected with inactive neurotrypsin. When wild-type neurotrypsin was cotransfected with agrin, no agrin signal was found in the cell lysate. However, new bands of 110, 90, and 22 kDa were found in the culture supernatant. None of these agrin fragments were found in the supernatants of cotransfections with the inactive form of neurotrypsin. Together, these results demonstrated that proteolytically active neurotrypsin cleaved agrin, resulting in the release of three C-terminal fragments into the culture supernatant.

### Example 2: The two neurotrypsin-dependent cleavage sites of agrin are homologous and evolutionarily conserved

To determine the neurotrypsin-dependent cleavage sites of agrin, neurotrypsin was coexpressed in HEK293T cells with either a transmembrane full-length form of rat agrin (splice variant y4z8) or the C-terminal fragment of agrin comprising the LG2, EG4, and LG3 domain using PEI transfection (Baldi et al., 2005). After 3 days the supernatants were harvested. For the purification of the 90-kDa fragment, 11 supernatant of cells transfected with full-length agrin was loaded onto a heparin sepharose CL-6B column (GE Healthcare) equilibrated with 150 mM NaCl in 20 mM Tris-HCl, pH 7.5. Proteins were eluted in a gradient from 150 mM to 1 M NaCl in 20 mM Tris-HCl, pH 7.5. The 22-kDa fragment was purified via its C-terminal StrepTag from 24 ml medium of cells transfected with the C-terminal fragment of agrin with a StrepTactin column (IBA) according to the manufacturer's recommendation. For N-terminal sequencing by Edman degradation, the peptide samples were separated on 4-12% NuPAGE gels, electrotransferred onto a PVDF membrane and analyzed on a Procise 492 cLC Sequencer (Applied Biosystems) at the Functional Genomics Center Zurich.

This way, the N-terminal sequence ASCYN SPLGCCSDGK (SEQ ID NO: 5) was found for the 90-kDa fragment and SVGDLETLAF (SEQ ID NO: 6) for the 22-kDa fragment. Therefore, one cleavage site is located between the first S/T-segment and the SEA domain, C-terminally of Arg₉₉₅ in the sequence PIER-ASCY (SEQ ID NO: 7, Fig. 3). The second cleavage site was localized between the fourth EGF-like and the LG3 domain, C-terminally of Lys₁₇₅₄ in the sequence LVEK-SVGD (SEQ ID NO: 8, Fig. 3). Amino acid numbers refer to membrane-anchored rat agrin (P25304; splice variant x4y0). We termed the scissile bond between R₉₉₅ and A₉₉₆ as α-cleavage site and the scissile bond between K₁₇₅₄ and S₁₇₅₅ as β-cleavage site.

An alignment (Table 1) of the two cleavage sequences of rat agrin with corresponding segments of various vertebrate species showed a stringent conservation of the amino acids flanking the cleavage sites.

**Table 1**

| | α site | | | | | | | | | | β site | | | | | | | | | | | |
|---|---|---|---|---|---|---|---|---|---|---|---|---|---|---|---|---|---|---|---|---|---|---|
| Species | P5 | P4 | P3 | P2 | P1 | ↓ | P1' | P2' | P3' | P4' | P5' | P5 | P4 | P3 | P2 | P1 | ↓ | P1' | P2' | P3' | P4' | P5' |
| Homo sapiens | P | P | V | E | R | ↓ | A | S | C | Y | N | G | L | V | E | K | ↓ | S | A | G | D | V |
| Pan troglodytes | P | P | V | E | R | ↓ | A | S | C | Y | N | G | L | V | E | K | ↓ | S | A | G | D | V |
| Bos taurus | L | P | M | E | R | ↓ | A | S | C | Y | N | G | L | I | E | K | ↓ | S | A | G | D | L |
| Canis familiaris | P | P | M | E | R | ↓ | A | S | C | Y | N | G | L | I | E | K | ↓ | S | A | G | D | V |
| Rattus norvegicus | P | P | I | E | R | ↓ | A | S | C | Y | N | G | L | V | E | K | ↓ | S | V | G | D | L |
| Mus musculus | P | P | I | E | R | ↓ | A | S | C | Y | N | G | I | V | E | K | ↓ | S | V | G | D | L |
| Gallus gallus | P | A | I | E | R | ↓ | A | T | C | Y | N | V | I | I | E | K | ↓ | A | A | G | D | A |
| Discopyge ommata | V | P | N | E | R | ↓ | S | T | C | D | N | A | L | E | E | K | ↓ | S | A | S | G | S |
| Rana pipiens | | | | | | | | | | | | A | T | I | E | K | ↓ | S | A | G | S | S |
| Danio rerio | | | | | | | | | | | | T | I | F | E | K | ↓ | S | A | G | D | T |
| Consensus | P | P | I | E | R | ↓ | A | S | C | Y | N | G | L | V | E | K | ↓ | S | A | G | D | V |
| | L | A | M | | | ↓ | S | T | | D | | A | I | I | | | ↓ | A | V | S | G | L |
| | V | | V | | | ↓ | | | | | | V | T | E | | | ↓ | | | | S | S |
| | | | N | | | ↓ | | | | | | T | | F | | | ↓ | | | | | T |
| | | | | | | | | | | | | | | | | | ↓ | | | | | A |

Alignment of agrin sequences of different species. For comparison of the amino acid residues surrounding the scissile bond of the α- and β-cleavage sites, we used the terminology of Schechter and Berger (1967). P1 denotes the N-terminal and P1' the C-terminal residue engaged in the scissile bond. Flanking residues in the N-terminal direction are denoted P2, P3, ... Pn, flanking residues in the C-terminal direction are denoted P2', P3', ... Pn'.

As is apparent from Table 1, the α site had a strictly conserved Arg at P1 and a Glu at P2. The β site had a strictly conserved Lys at P1 and a Glu at P2. The residues at the P3 position were more variable, while predominantly apolar residues were found at P4 of both sites. On the C-terminal side of the scissile bond, the P1' and P2' residues are well conserved at each site, with predominantly an Ala-Ser sequence at the α and a Ser-Ala sequence at the β site. Well conserved but distinct residues were found at P3' and P4' of the two sites.

In summary, the consensus sequences of the α and β site exhibit an identical profile, with a strict conservation of the P1 and P2 residue, a variable occupation of the P3 position, and a high degree of conservation at P4, P1', P2', and P3'. The gross profile of the individual consensus sequences of the α and β sites is maintained when a combined consensus sequence of the two cleavage sites is generated (Table 2), confirming the homology between the individual cleavage sequences at the α and β site.

**Table 2 Consensus sequence combining the α- and β-cleavage sites.**

| | P5 | P4 | P3 | P2 | P1 | ↓ | P1' | P2' | P3' | P4' | P5' |
|---|---|---|---|---|---|---|---|---|---|---|---|
| combined consensus P | P | P | I | E | R | ↓ | S | A | G | D | N |
| | G | L | V | | K | ↓ | A | S | C | Y | V |
| | A | I | M | | | ↓ | | T | S | G | L |
| | V | A | N | | | ↓ | | V | | S | S |
| | T | T | E | | | ↓ | | | | | T |
| | L | | F | | | ↓ | | | | | A |

### Example 3: Neurotrypsin-dependent cleavage of agrin occurs in vivo and is not found in neurotrypsin-deficient mice

We investigated different tissues from wild-type and neurotrypsin-deficient mice for the presence of agrin fragments. Brain, lung, and kidney were isolated from 10-day-old wild-type and neurotrypsin-deficient mice. Approximately 1 g of each tissue was homogenized with a Potter homogenizer in 1 ml lysis buffer (320 mM sucrose, 5 mM HEPES, pH 7.4) containing a cocktail of protease inhibitors (Sigma). Cell lysates (40 µg kidney, 80 µg brain/lung) were analyzed for neurotrypsin expression and agrin cleavage using immunoblotting. β-Actin was probed on the stripped membrane of the agrin immunoblot. As shown in Fig. 4, strong immunoreactivity for full-length agrin was detected in brain, kidney, and lung of wild-type mice. In these tissues we also found the 90-kDa cleavage product of agrin. The 22-kDa cleavage product was readily detectable in brain and kidney, but it was absent in the lung. In neurotrypsin-deficient mice no agrin immunoreactivity was detectable at 22 kDa or 90 kDa. These results demonstrate that agrin cleavage by neurotrypsin occurs *in vivo* and, furthermore, the absence of cleavage products in neurotrypsin-deficient mice indicates that agrin cleavage strictly depends on neurotrypsin.

### Example 4: Induction of long-term potentiation (LTP) results in increased proteolytic activity of neurotrypsin in CNS tissue, resulting in the generation of agrin fragments, including the 22-kDa fragment.

The effect of a learning-like neural activity on the proteolytic activity of neurotrypsin is demonstrated by the induction of long-term potentiation (LTP) in tissue slices of the CA1 region of the hippocampus. At present, LTP is the most widely accepted correlate of learning and memory at the cellular, molecular, and electrophysiological level (Martin et al., 2000). To maximize the number of synapses participating in LTP, a chemical protocol for LTP induction (Otmakhov et al., 2004) was used with a combination of picrotoxin (50 µM), forskolin (50 µM), and rolipram (0.1 µM). The combination of these compounds induces strong LTP lasting for several hours without requiring any electrical stimulation.

To prepare the hippocampal slices, the hippocampus, together with the adjacent cerebral cortex, is rapidly dissected from 4-5 weeks-old C57BL/6 mice, then cut vertically to the long axis of the hippocampus into 400 µm thick slices using a McIlwain mechanical tissue chopper (The Mickle Laboratory Engineering Co. Ltd.). The slices are transferred into artificial cerebrospinal fluid (ACSF) without calcium (120 mM NaCl, 3 mM KCl, 1.2 mM NaH₂PO₄, 23 mM NaHCO₃, 11 mM glucose, 2.4 mM MgCl₂) oxygenated by 95% O₂ / 5% CO₂ and incubated for one hour at room temperature, in order to provide sufficient time for the brain tissue to recover from the dissection injury. Following a pre-incubation, the slices are incubated in ACSF with calcium for 20 minutes (120 mM NaCl, 3 mM KCl, 1.2 mM NaH₂PO₄, 23 mM NaHCO₃ 11 mM glucose, 4 mM CaCl₂). Chemical LTP is then induced in the slice by incubation with a combination of 50 µM picrotoxin, 50 µM forskolin, and 0.1 µM rolipram (PFR stimulation) for 16 minutes.

To examine the proteolytic activity of neurotrypsin in slices, the proteolytic processing of its substrate, agrin, is assessed by Western blotting. Immediately after the PFR stimulation, the slices are homogenized in 1% Triton X-100, 0.32 M sucrose, 0.5 mM EDTA, 5 mM HEPES, pH 7.4. Debris are removed by centrifugation (16,000×g for 30 minutes at 4°C). The proteins of the supernatant (75 µg of protein) are electrophoretically separated in 10% SDS polyacrylamide gel, then electrotransferred to a PVDF membrane. Subsequently, the membrane is immersed into methanol for 10 seconds and dried for blocking. Following blocking, the membrane is incubated with an affinity-purified rabbit anti-agrin polyclonal antibody (R132; 1 µg/ml) diluted in Tris-buffered saline (TBS; 0.15 M NaCl, 10 mM Tris-HCl, pH 8.0,) containing 0.1% Tween-20 (TTBS). R132 recognizes the middle, 90-kDa fragment of agrin that is generated by cleavage of agrin at the α- and β-site by neurotrypsin. After washing 3 times for 10 minutes in TTBS, the membrane is incubated overnight at 4°C in TTBS containing an anti-rabbit IgG antibody conjugated with peroxidase (Sigma; diluted 1:10,000), then washed 3 times in TTBS and once in TBS. Subsequently the membrane is immersed in a chemiluminescent substrate (CHEMIGLOW; Alpha Innotech Corporation) for 5 minutes to visualize the agrin fragment. The chemiluminescent signal was detected with a ChemiImager (Alpha Biotech).

As shown in Figure 5A, the amount of the neurotrypsin-dependent 90-kDa agrin fragment is increased by PFR stimulation (PFR) compared to the non-stimulated control (No Stim). This increase is statistically significant, as determined by the Student's t-test (3 independent blotting experiments using different samples; p < 0.05; Figure 5B). This result indicates that the PFR stimulation induces an increase in neurotrypsin-dependent proteolytic activity. The increase in the amount of the 90-kDa fragment of agrin also indicates an increase of the 22-kDa fragment, the C-terminal fragment of agrin, because the 90-kDa fragment of agrin cannot be generated without the generation of 22-kDa fragment.

### Example 5: Activity-induced increase of the proteolytic activity of neurotrypsin and the resulting generation of the 22-kDa fragment of agrin in CNS tissue results in an increased number of dendritic filopodia.

To enable the investigation of dendritic filopodia, a transgenic mouse line expressing green fluorescent protein (GFP) or membrane-targeted green fluorescence protein (mGFP) in single neurons under the control of the Thy-1 promoter is crossbred with a neurotrypsin-deficient mouse line. Transgenic mice expressing GFP or mGFP are generated as described previously (Feng et al., 2000; De Paola et al., 2003). Neurotrypsin-deficient mice expressing GFP or mGFP are generated by crossing neurotrypsin-deficient mice with transgenic mice expressing GFP or mGFP in single neurons. Homozygeous neurotrypin-deficient mice overexpressing mGFP and the corresponding mGFP-overexpressing wild-type mice are used in the following analysis at the age of 5-6 weeks.

Acute hippocampal slices are prepared and stimulated by PFR as described in Example 4. Immediately after the stimulation period, the slices are fixed by incubation in 4% paraformaldehyde, 4% sucrose, 0.1 M phosphate-buffered saline (PBS), pH 7.4, overnight at 4°C. After washing in PBS, the slices are mounted on slides and cover-slipped in Vectashield mounting medium (Vector Laboratory Inc.). The fluorescent signals in the secondary apical dendrites of hippocampal CA1 pyramidal neurons expressing mGFP are observed using a confocal microscope (Leica), and serial images at intervals of 0.1221 µm are collected. From these, three-dimensional images are reconstructed using the Surpass Volume mode in the Imaris imaging software (Bitplane AG). The number of typical filopodia are manually counted over a length of 30-40 µm along 12-20 independent dendrites by inspecting the three-dimensional images from all directions. Dendritic filopodia are identified according to the following morphological criteria: 1) a dendritic membrane protrusion is categorized as a filopodium if its length is at least twice the average length of the spines on the same dendrite; 2) the ratio of the head diameter to the neck diameter is smaller than 1.2:1, and 3) the ratio of the filopodial length to the neck diameter is larger than 3:1 (Grutzendler et al., 2002).

The results indicate that, under the PFR stimulation, the number of dendritic filopodia in the hippocampus of wild-type (WT) mice is increased in comparison to non-stimulated controls. Statistical analysis using data from 12-20 independent dendrites shows a significant increase in the number of filopodia by the PFR stimulation (p < 0.01 by Student's t-test; Figure 6). In contrast, chemical stimulation by PFR does not alter the number of filopodia in neurotrypsin-deficient (ntd) mice (Figure 6). This indicates that neurotrypsin is required for the activity-driven increase in filopodia number. In summary, these results indicate that increased proteolytic activity of neurotrypsin in CNS neurons results in an increased number of dendritic filopodia.

### Example 6: Administration of the 22-kDa fragment of agrin to CNS neurons results in an increased number of dendritic filopodia.

The 22-kDa fragment, the C-terminal fragment of agrin, is generated naturally by the proteolytic activity of neurotrypsin at the β-cleavage site of agrin. Recombinant 22-kDa fragment of agrin is produced as described in Example 7). To investigate whether 22-kDa fragment generation by neurotrypsin is involved in the activity-driven increase in dendritic filopodia number the filopodia along the dendrites of hippocampal CA1 neurons are counted in 22-kDa fragment-treated acute slices from homozygeous neurotrypsin-deficient mice expressing GFP or mGFP in single neurons. Neurotrypsin-deficient mice are generated as described in Example 9. Transgenic mice expressing GFP or mGFP are generated as described previously (Feng et al., 2000; De Paola et al., 2003). Neurotrypsin-deficient mice expressing GFP or mGFP are generated by crossing neurotrypsin-deficient mice with transgenic mice expressing GFP or mGFP in single neurons. Acute hippocampal slices are prepared from neurotrypsin-deficient, mGFP-overexpressing mice as described in Example 4. These slices are divided in 4 groups in order to subject them to 4 different stimulation conditions: no stimulation (No Stim), incubation with PFR (PFR), incubation with both 22-kDa fragment and PFR (22-kDa frag + PFR), and incubation with 22-kDa fragment (22-kDa frag). The experimental conditions designated 22-kDa frag and 22-kDa frag + PFR contain human, rat, or mouse 22-kDa fragment (prepared as described in Example 7) at a concentration of 22 nM in oxygenated ACSF. Immediately after the stimulation period (16 minutes), the slices are fixed, mounted on slides, and serial images at 0.1221 µm are taken from secondary apical dendrites of neurons expressing GFP by confocal microscopy as described in Example 5. Filopodia are counted in 14-20 secondary apical dendrites (each 30-40 µm long) of CA1 pyramidal neurons after reconstruction of three-dimensional images with the Imaris imaging software.

As shown in Figure 7, the increase the number of dendritic filopodia upon PFR stimulation is lost in neurotrypsin-deficient mice (this effect was also shown in Example 5 and in Figure 6). However, the addition of 22-kDa fragment to PFR reestablishes the increase in filopodia number in hippocampal slices from neurotrypsin-deficient mice to the same level as found with PFR in wild-type mice (p < 0.001 by Student's t-test). This demonstrates that activity-driven increase in filopodia is dependent on the generation of 22-kDa fragment by neurotrypsin-dependent proteolytic cleavage form agrin (which does not occur in neurotrypsin-deficient mice). Furthermore, administration of 22 nM purified recombinant human 22-kDa fragment alone (without PFR) induces a significant increase in filopodia in the absence of chemical LTP induction by PFR. The filopodia-inducing effect of the 22-kDa fragment is also observed in the absence of the LTP-inducing stimulus, indicating that the 22-kDa fragment is a downstream mediator of the filopodia-inducing effect of LTP. In short, these results indicate that the 22-kDa fragment acts as a filopodia-inducer.

### Example 7: Cloning, expression and purification of the 22-kDa C-terminal fragment of human agrin.

The BAC DNA with accession number BC007649, having the 3' region of the cDNA of human agrin as insert, is used to generate a DNA fragment coding for the last LG domain of agrin by PCR amplification. The PCR primers used are
(SEQ ID NO: 9) 5'-GGG CGA **GTT AAC** CAC CAT CAC CAT CAC CAT CAC CAT TCA GCG GGG GAC GTG GAT ACC TTG GC-3', introducing a HpaI site (5' humanC), and
(SEQ ID NO: 10) 5'-TTA CCT **GCG GCC GCT** CAT GGG GTG GGG CAG GGC CGC AGC TC -3', introducing a NotI site (3' humanC).

Using this strategy, the DNA sequence which encodes an N-terminal 8xHis tag is inserted. The resulting PCR product is cleaved with the restriction enzymes NotI and HpaI (boldface in the primer sequences) and cloned into the pEAK8 vector containing the coding sequence for the signal peptide of human calsyntenin-1 cut with the same restriction enzymes. The resulting construct pEAK8-22-kDa fragment contains the coding region of the signal sequence of human calsyntenin-1 as a secretion signal for 22-kDa fragment. Cloning as well as amplification of the plasmid is performed in *E*. *coli.* For eucaryotic protein synthesis HEK 293T cells are transfected using the calcium phosphate method. During translation in HEK 293T cells the signal peptide is cleaved off. The resulting secreted protein has a sequence wherein the leader sequence ARVNHHHHHH HH (SEQ ID NO:11) is connected to the N-terminus of the sequence obtained by neurotrypsin-dependent agrin cleavage at the β site comprising the LG 3 domain and the C-terminus of agrin of SEQ ID NO:12.

This polypeptide has a molecular mass of approximately 20 kDa without the N-terminal ARVN-8xHis tag. The total mass including the tag is about 21.5 kDa.

For protein production, HEK 293T cells are cultivated to 80% confluency in seven culture plates of 500 cm² (Corning) with 100 ml DMEM medium (GIBCO) supplemented with 10% FCS each. For transfection 35 ml 500 mM CaCl₂ and 35 ml HBS buffer (50 mM HEPES, 140 mM NaCl, 1.5 mM Na₂HPO₄, pH 7.1) are equilibrated to room temperature. Two mg DNA of the pEAK8-22-kDa fragment expression construct are added to the CaCl₂ solution and mixed with the HBS buffer. The transfection mixture is incubated at room temperature for 30 min. For transfection of a 500 cm² plate of HEK cells 10 ml of the transfection mixture are added dropwise to the culture and incubated for 4 h at 37°C. The transfection mixture is then removed by washing once with PBS and addition of DMEM medium without FCS. After 60 h the conditioned medium is harvested and filtered using a Steritop 0.22 µm filter (Millipore). The supernatant is dialysed against 20 mM Tris-HCl, 400 mM NaCl, pH 8.5, and submitted to IMAC purification using a Ni-NTA column (10 ml HisSelect, Sigma-Aldrich) on a BioLogic liquid chromatography system (Biorad). The conditioned and dialyzed medium is loaded with a flow rate of 5 ml/min, the column is washed with 20 CV of 20 mM Tris-HCl, 400 mM NaCl, pH 8.5. For elution a linear gradient from 0 to 250 mM imidazole in washing buffer for 10 column volumes is used. Fractions containing the pure recombinant 22-kDa fragment are pooled, concentrated with a 15 ml spin concentrator (Millipore) and the buffer is exchanged with a NAP 25 column (Pharmacia) to 10 mM MOPS, 100 mM NaCl, pH 7.5. The purified protein is frozen in liquid nitrogen and stored at -80°C. The 22-kDa fragment generated this way contains an N-terminal 8xHis tag followed by the P' residues of the cleavage site β and the natural C-terminus of agrin.

Similarly, a recombinant 22-kDa fragment of rat or mouse agrin comprising the same domain structure is cloned, expressed, and purified.

It is also possible to construct a 22-kDa fragment with other tags or a tag-free variant of 22-kDa fragment with standard cloning techniques. Expression is achieved in standard eukaryotic or prokaryotic cell systems and purification of the desired protein is achieved either by standard liquid chromatography or by refolding from inclusion bodies.

To generate a 22-kDa fragment which is identical to the form found *in vivo,* the N-terminal His-tag is omitted and 22-kDa fragment is generated from any kind of cDNA encoding a C-terminal fragment of agrin comprising the LG3 domain and the cleavage site β of agrin (e.g. full-length agrin or the 44-kDa C-terminal fragment) by digestion with neurotrypsin *in vitro* or during expression of agrin or the 44-kDa agrin fragment. The 22-kDa fragment is purified as described above.

### Example 8: Generation of polyclonal antibodies against the 22-kDa fragment of agrin.

Polyclonal antibodies against the LG3 domain of agrin are generated by immunizing rabbits with 50 µg rat agrin LG3 domain. The resultant antibody is useful for the detection of human, mouse or rat full-length agrin, as well as for the detection of agrin fragments containing the LG3 domain of agrin.

### Example 9: The 22-kDa fragment of agrin can be detected in brain homogenate and blood of the mouse

We investigated the occurrence of the agrin fragments in the soluble fraction of brain homogenates and in the blood serum of the mouse. For detection we used specific, affinity-purified antibodies generated in our laboratory against the 90-kDa and the 22-kDa fragment. The 22-kDa fragment could be readily detected on Western blots of both brain homogenates and blood serum (Fig. 8). The included brain homogenates of homozygeous neurotrypsin-deficient mice (-/- in Fig. 8, A and B) did not contain the 22-kDa fragment, whereas the heterozygous form of neurotrypsin-deficient mice (+/- in Fig. 8B) showed the 22-kDa fragment at a reduced level. Together, these results indicate that the 22-kDa fragment detected by Western blotting is due to the action of neurotrypsin and that it can be detected in the soluble fraction of the brain homogenate as well as in the blood serum.

### Example 10: The 22-kDa fragment of agrin is found in human CSF

We also tested human samples, i.e. urine, blood, and cerebrospinal fluid for the presence of neurotrypsin-dependent agrin fragments. So far, we did not find any evidence for the presence of the 110-kDa or the 90-kDa fragment in any of the body fluids. However, we found the 22-kDa fragment in both the cerebrospinal fluid and the blood serum.

As shown in Fig. 9A, an immunoreactive signal for the 22-kDa fragment of agrin was found at all ages tested, ranging from a 1 month-old child, through further developmental stages and adulthood, to the CSF of an 86-year-old woman. As expected based on the temporal expression pattern of neurotrypsin with a peak expression during early postnatal development, we found the highest amount of the 22-kDa fragment of agrin in the CSF of 2- and 9-month-old children. Interestingly, a clearly elevated level of the 22-kDa fragment was found in the 86-year-old woman. However, definitely more samples will have to be measured in order to draw conclusions to the biological or pathophysiological relevance of this observation.

To verify the identity of the 22-kDa immunoreactive band, we isolated the underlying protein from cerebrospinal fluid and subjected it to an LC/ESI/MS/MS analysis. For the purpose of identifying the protein unequivocally based on its partial peptide sequence, it was fragmented by a tryptic digestion. The resulting fragments were then separated by liquid chromatography and subjected to an MS/MS analysis. As shown in Fig. 9B, two peptides exactly matching the amino acid sequence of the first 27 amino acids (peptide #1 comprising 13 amino acids (SEQ ID NO: 3); peptid #2 comprising 14 amino acids (SEQ ID NO: 4)) of the 22-kDa fragment of human agrin were detected. Both peptides end with a basic amino acid, confirming their tryptic nature. Peptide #1 starts at the neurotrypsin-dependent P cleavage site of agrin. No peptide located outside of the margins of the 22kDa fragment of agrin was found. Together, these results confirm the identity of the immunoreactive 22-kDa band found in Western blots as the neurotrypsin-dependent 22-kDa fragment of agrin. These results indicate CSF as a reporter compartment for monitoring the activity of neurotrypsin in the brain.

### Example 11: The 22-kDa fragment of agrin can be detected in human blood

The search for neurotrypsin-dependent fragments of agrin was also extended to extraneural samples. As shown in Fig. 10, Western blotting of human blood serum showed a clear immunoreactive band. However, the signal detected in blood serum was less intensive as that found in CSF. For unequivocal detection, a chromatographic separation and differential enrichment of the serum proteins was required to reveal a signal (arrow in Fig. 10). Currently ongoing work is aimed at the structural validation of the identity of the immunoreactive 22-kDa band as a derivative of agrin. Successful confirmation provided, the blood serum will also be used as a reporter compartment for monitoring neurotrypsin *in vivo.* Quantitative data on the 22-kDa fragment in blood serum will allow drawing conclusions of neurotrypsin activity at the neuromuscular junction and in extraneural tissue, such as the kidney and the lung.

### REFERENCES:

Aimes RT, Zijlstra A, Hooper JD, Ogbourne SM, Sit ML, Fuchs S, Gotley DC, Quigley JP, Antalis TM (2003) Endothelial cell serine proteases expressed during vascular morphogenesis and angiogenesis. Thrombosis and haemostasis 89: 561-572
Baldi L, Muller N, Picasso S, Jacquet R, Girard P, Thanh HP, Derow E, Wurm FM (2005) Transient gene expression in suspension HEK-293 cells: application to large-scale protein production. Biotechnology progress 21: 148-153
Bezakova G, Ruegg MA (2003) New insights into the roles of agrin. Nature reviews 4: 295-308
De Paola V, Arber S, Caroni P (2003) AMPA receptors regulate dynamic equilibrium of presynaptic terminals in mature hippocampal networks. Nature neuroscience 6: 491-500
Feng G, Mellor RH, Bernstein M, Keller-Peck C, Nguyen QT, Wallace M, Nerbonne JM, Lichtman JW, Sanes JR (2000) Imaging neuronal subsets in transgenic mice expressing multiple spectral variants of GFP. Neuron 28: 41-51
Gill, D. S., and Damle, N. K. (2006). Biopharmaceutical drug discovery using novel protein scaffolds. Curr Opin Biotechnol 17, 653-658.
Grutzendler J, Kasthuri N, Gan WB (2002) Long-term dendritic spine stability in the adult cortex. Nature 420: 812-816
Gschwend TP, Krueger SR, Kozlov SV, Wolfer DP, Sonderegger P (1997) Neurotrypsin, a novel multidomain serine protease expressed in the nervous system. Molecular and cellular neurosciences 9: 207-219
Martin SJ, Grimwood PD, Morris RG (2000) Synaptic plasticity and memory: an evaluation of the hypothesis. Annual review of neuroscience 23: 649-711
Molinari F, Rio M, Meskenaite V, Encha-Razavi F, Auge J, Bacq D, Briault S, Vekemans M, Munnich A, Attie-Bitach T, Sonderegger P, Colleaux L (2002) Truncating neurotrypsin mutation in autosomal recessive nonsyndromic mental retardation. Science (New York, NY 298: 1779-1781
Otmakhov N, Khibnik L, Otmakhova N, Carpenter S, Riahi S, Asrican B, Lisman J (2004) Forskolin-induced LTP in the CA1 hippocampal region is NMDA receptor dependent. Journal of neurophysiology 91: 1955-1962
Proba K, Gschwend TP, Sonderegger P (1998) Cloning and sequencing of the cDNA encoding human neurotrypsin. Biochimica et biophysica acta 1396: 143-147
Roque, A. C., Lowe, C. R., and Taipa, M. A. (2004). Antibodies and genetically engineered related molecules: production and purification. Biotechnol Prog 20, 639-654.
Sanes JR, Lichtman JW (2001) Induction, assembly, maturation and maintenance of a postsynaptic apparatus. Nat Rev Neurosci 2: 791-805
Schechter I, Berger A (1967) On the size of the active site in proteases. I. Papain. Biochemical and biophysical research communications 27: 157-162

## Claims

1. Method for the detection of the in-vivo activity of neurotrypsin wherein the amount of the 22-kDa-fragment of agrin is measured in a sample taken from a patient and the measured amount of the 22-kDa-fragment of agrin in the sample is used to calculate the activity of neurotrypsin with the sample being blood or urine.

2. Method according to claim 1, in which a 22-kDa-fragment of agrin prepared by recombinant techniques is used as a reference.

3. Use of the method according to claim 1 for diagnosis and monitoring of neurotrypsin-related disturbances.

4. Use according to claim 3 for diagnosis and monitoring of diseases caused by deregulation of neurotrypsin.

5. Use according to claim 3 or 4 for diagnosis and monitoring of neurotrypsin-related diseases and disturbances of the neural and the neuromuscular system.

6. Use according to claim 3 or 4 for diagnosis and monitoring of neurotrypsin-related diseases and disturbances of non-neural systems, especially the kidney and the lung.

7. Use of the method according to claim 1 in clinical or preclinical studies to establish the effect of substances on the activity of neurotrypsin.

8. Use of the 22-kDa-fragment of agrin, detected in blood or urine in a sample taken from a patient, as biomarker for neurotrypsin-related disturbances.

9. Use according to claim 8 for diagnosis and monitoring of diseases caused by deregulation of neurotrypsin.

10. Use according to claim 8 or 9 for diagnosis and monitoring of neurotrypsin-related diseases and disturbances of the neuronal and the neuromuscular system.

11. Use according to claim 8 or 9 for diagnosis and monitoring of neurotrypsin-related diseases and disturbances of non-neural systems.

12. Use of the 22-kDa-fragment of agrin, detected in blood or urine in a sample taken from a patient, as biomarker in clinical or preclinical studies to establish the effect of substances on the activity of neurotrypsin.

## Patentansprüche

1. Verfahren zur Bestimmung der in-vivo-Aktivität von Neurotrypsin, bei dem der Gehalt des 22-kDa-Fragmentes von Agrin in einer einem Patienten entnommenen Probe gemessen wird, und der gemessene Gehalt des 22-kDa-Fragmentes von Agrin in der Probe verwendet wird, um die Neurotrypsin-Aktivität zu berechnen, wobei die Probe Blut oder Urin ist.

2. Verfahren nach Anspruch 1, bei dem ein rekombinant hergestelltes 22-kDa-Fragment von Agrin als Referenz verwendet wird.

3. Verwendung des Verfahrens nach Anspruch 1 zur Diagnose und Überwachung von mit Neurotrypsin in Beziehung stehenden Störungen.

4. Verwendung nach Anspruch 3 zur Diagnose und Überwachung von Krankheiten, die durch eine Deregulierung von Neurotrypsin hervorgerufen werden.

5. Verwendung nach Anspruch 3 oder 4, zur Diagnose und Überwachung von mit Neurotrypsin in Beziehung stehenden Erkrankungen und Störungen des neuralen und neuromuskulären Systems.

6. Verwendung nach Anspruch 3 oder 4 zur Diagnose und Überwachung von mit Neurotrypsin in Beziehung stehenden Erkrankungen und Störungen des nicht-neuralen Systems, insbesondere der Niere und der Lunge.

7. Verwendung eines Verfahrens nach Anspruch 1 in klinischen oder präklinischen Studien zur Bestimmung der Wirkung von Substanzen auf die Neurotrypsin-Aktivität.

8. Verwendung des 22-kDa-Fragmentes von Agrin, bestimmt im Blut oder Urin in einer dem Patienten entnommenen Probe, als Biomarker für mit Neurotrypsin in Beziehung stehenden Störungen.

9. Verwendung nach Anspruch 8 zur Diagnose und Überwachung von Krankheiten, die durch eine Deregulierung des Neurotrypsin hervorgerufen werden.

10. Verwendung nach Anspruch 8 oder 9 zur Diagnose und Überwachung von mit Neurotrypsin in Beziehung stehenden Erkrankungen und Störungen des neuronalen und des neuromuskulären Systems.

11. Verwendung nach Anspruch 8 oder 9 zur Diagnose und Überwachung von mit Neurotrypsin in Beziehung stehenden Krankheiten und Störungen des nicht-neuralen Systems.

12. Verwendung des 22-kDa-Fragmentes von Agrin, bestimmt in Blut oder Urin in einer einem Patienten entnommenen Probe, als Biomarker in klinischen oder präklinischen Studien zur Bestimmung der Wirkung von Substanzen auf die Neurotrypsin-Aktivität.

## Revendications

1. Procédé de détection de l'activité in vivo de la neurotrypsine, la quantité de fragment d'agrine de 22 kDa étant mesurée dans un échantillon prélevé sur un patient et la quantité de fragment d'agrine de 22 kDa mesurée dans l'échantillon étant utilisée pour calculer l'activité de la neurotrypsin, l'échantillon étant un échantillon de sang ou d'urine.

2. Procédé selon la revendication 1 pour lequel un fragment d'agrine de 22 kDa produit par des techniques recombinantes est utilisé comme référence.

3. Utilisation du procédé selon la revendication 1 pour le diagnostic et le suivi de troubles liés à la neurotrypsine.

4. Utilisation selon la revendication 3 pour le diagnostic et le suivi de maladies causées par une dérégulation de la neurotrypsine.

5. Utilisation selon les revendications 3 ou 4 pour le diagnostic et le suivi de maladies et de troubles du système neural et neuromusculaire liées à la neurotrypsine.

6. Utilisation selon les revendications 3 ou 4 pour le diagnostic et le suivi de maladies et de troubles de systèmes non neuraux liées à la neurotrypsine, plus particulièrement du rein et du poumon.

7. Utilisation du procédé selon la revendication 1 dans le cadre d'études cliniques et pré-cliniques pour établir l'effet de substances sur l'activité de la neurotrypsine.

8. Utilisation du fragment d'agrine de 22 kDa décelé dans le sang ou l'urine d'un échantillon prélevé sur un patient comme biomarqueur de troubles liés à la neurotrypsine.

9. Utilisation selon la revendication 8 pour le diagnostic et le suivi de maladies causées par une dérégulation de la neurotrypsine.

10. Utilisation selon les revendications 8 ou 9 pour le diagnostic et le suivi de maladies et de troubles du système neuronal et neuromusculaire liées à la neurotrypsine.

11. Utilisation selon les revendications 8 ou 9 pour le diagnostic et le suivi de maladies et de troubles de systèmes non neuraux liées à la neurotrypsine.

12. Utilisation du fragment d'agrine de 22 kDa décelé dans le sang ou l'urine d'un échantillon prélevé sur un patient comme biomarqueur dans le cadre d'études cliniques et pré-cliniques pour établir l'effet de substances sur l'activité de la neurotrypsine.
